## Europäisches Patentamt

## European Patent Office

## Office européen des brevets

(11) Veröffentlichungsnummer: **0 321 597 B1**

(12) **EUROPÄISCHE PATENTSCHRIFT**

(45) Veröffentlichungstag der Patentschrift: **11.11.92**

(51) Int. Cl.⁵: **B01J 20/22**, B01D 15/00, A61M 1/36, A61K 35/16

(21) Anmeldenummer: **87119017.9**

(22) Anmeldetag: **22.12.87**

(54) **Selektives Adsorbens zur Bindung von Lipoproteinen niedriger Dichte.**

(43) Veröffentlichungstag der Anmeldung:
**28.06.89 Patentblatt  89/26**

(45) Bekanntmachung des Hinweises auf die Patenterteilung:
**11.11.92 Patentblatt  92/46**

(84) Benannte Vertragsstaaten:
**AT BE CH DE ES FR GB IT LI LU NL SE**

(56) Entgegenhaltungen:
**EP-A- 0 165 709**
**EP-A- 0 225 867**
**GB-A- 2 000 150**
**GB-A- 2 101 906**

(73) Patentinhaber: **FRAUNHOFER-GESELLSCHAFT ZUR FÖRDERUNG DER ANGEWANDTEN FORSCHUNG E.V.**
**Leonrodstrasse 54**
**W-8000 München 19(DE)**

(72) Erfinder: **Loth, Fritz, Dr.**
**Ernst Thälmann-Str. 211**
**DD-1530 Teltow(DE)**
Erfinder: **Behm, Erasmus, Dr.**
**Walter Stöcker-Str. 27**
**DD-2510 Rostock(DE)**
Erfinder: **Toewe, Dagmar**
**Heinrich v. Kleist-Weg 18**
**DD-2540 Rostock(DE)**
Erfinder: **Klinkmann, Horst, Prof.Dr.**
**Balecke-Strasse 7a**
**DD-2500 Rostock(DE)**
Erfinder: **Ernst, Bruno, Dr.**
**Schliemannstr. 31**
**DD-2500 Rostock(DE)**
Erfinder: **Bertram, Dieter, Dr.**
**Str. d. 18. Oktober 8/75**
**DD-7010 Leipzig(DE)**

**Beschreibung**

Die Erfindung betrifft ein Verfahren sowie ein Adsorbens für die selektive Abtrennung von Lipoproteinen niedriger Dichte aus Blutplasma, die insbesondere im Bereich der Medizin und Medizintechnik anwendbar sind.

Lipoproteine niedriger Dichte (LDL) spielen in der Pathogenese der Arteriosklerose eine entscheidende Rolle. Aus zahlreichen klinischen Studien ist bekannt, daß sich das Risiko einer Arteriosklerose mit zunehmendem LDL-Gehalt im Blutplasma erhöht, während zwischen der Höhe des Plasmaspiegels der Lipoproteine hoher Dichte (HDL) und dem Risiko der Entwicklung von Blutgefäßverkalkungen eine inverse Korrelation besteht. Wegen der stark erhöhten LDL-Werte des Plasmas bei Patienten, die an familiärer Hypercholesterinämie leiden, ist hier das Arterioseserisiko besonders hoch. Diätetische und medikamentöse Behandlungen zeigen in diesen Fällen nicht die gewünschten Erfolge. Deshalb sind für diese Patienten Verfahren, mit deren Hilfe es gelingt, das Blutplasma von LDL zu befreien, von größter Bedeutung. Bekanntlich wurden hierfür zunächst Plasmaaustauschverfahren sowie spezielle Plasmafiltrationstechniken angewandt, die jedoch den Nachteil besitzen, daß auch andere Plasmabestandteile, wie z. B. Immunglobuline und Gerinnungsfaktoren, entfernt werden, bzw., daß sie sehr teuer und aufwendig sind.

In jüngster Zeit wurden zur Entfernung von LDL aus dem Blutplasma selektive Adsorbentien entwickelt, die eine wesentliche Verbesserung der bekannten therapeutischen Verfahren ermöglichen. Dabei handelt es sich einerseits um spezifische Adsorbentien (Immunadsorbentien), die aus Agaroseperlen bestehen, an die kovalent gegen die LDL-Apoproteine gerichtete Antikörper gebunden sind (W. Stoffel, Ch. Bode, Selective Removal of Low Density Lipoproteins, in "Selective Plasma Component Removal", A.A. Pineda Ed., Futura Publishing Comp., Mount Kisco, New York, 1984), und andererseits um ionisch modifizierte polymere Träger, wie Z.B. Polyvinylalkohol-, Agarose- oder Celluloseperlen, die mit anionischen Polyelektrolyten, wie z.B. Heparin oder Dextransulfat, beschichtet sind (Y. Homma, Y. Mikami, H. Tamachi, N. Nakaya, H. Nakamura, G. Araki, Y. Goto, Comparison of Selectivity of LDL Removal by Double Filtration and Dextran-Sulfate Cellulose Column Plasmaphoresis, Atherosclerosis 60 (1986) 23-27, US-A-4 103 685). Während der Nachteil der Immunadsorbentien vor allem in der aufwendigen Gewinnung und Fixierung der Antikörper und dem dadurch bedingten hohen Preis der Produkte zu sehen ist, haben die ionisch modifizierten polymeren Träger den Nachteil, daß sie gleichzeitig auch zweiwertige Metallionen, wie z.B. $Ca^{2+}$ und $Mg^{2+}$, binden. Da diese bei therapeutischen Verfahren mit den genannten Adsorbentien wieder ersetzt werden müssen, kompliziert und verteuert dies die Behandlung.

Aufgabe der Erfindung ist es, Adsorbentien zur Entfernung von LDL aus Blutplasma, die leicht verfügbar und kostengünstig sind und allen sonstigen Anforderungen an die Eigenschaften derartiger Materialien gerecht werden, sowie ein Verfahren zur LDL-Abtrennung anzugeben. Dabei sollen unmodifizierte poröse polymere Adsorbentien angegeben werden, die geeignet sind, selektiv und mit hoher Kapazität LDL aus Blutplasma zu binden. Die Adsorbentien sollen regenerierbar und sterilisierbar sein sowie Kugelform und eine hohe mechanische Festigkeit besitzen, um als Packung in einer Säule ein gutes Fließverhalten des Blutes oder Blutplasmas durch die Säule zu gewährleisten. Weiterhin sollen die Adsorbentien gut blutverträglich sein, d.h. plasmatische Systeme nicht beeinträchtigen.

Die Aufgabe wird anspruchsgemäß gelöst. Vorteilhafte Ausführungsformen sind Gegenstand der abhängigen Ansprüche.

Das erfindungsgemäße selektive Adsorbens zur Bindung von LDL aus Blutplasma ist mechanisch stabile, kugelförmige, chemisch unmodifizierte, poröse Perlcellulose, die ein Gesamtporenvolumen von mindestens 2 ml/g Trockenmasse aufweist, wobei der Anteil an zugänglichen Makroporen mit einem Eingangsquerschnitt von 20 bis 2000 nm mindestens 10 % des Gesamtvolumens beträgt. Als Gesamtporenvolumen ist hierbei der Volumenanteil eines unpolaren, die Cellulose nicht quellenden Lösungsmittels definiert, der nach Verdrängung des Wassers aus der feuchten Perlcellulose und Abschleudern des anhaftenden Lösungsmittels im Hohlraumsystem der Perlcellulose zurückgehalten wird. Der Anteil an zugänglichen Makroporen mit einem bestimmten Eingangsquerschnitt ergibt sich aus der Penetrierbarkeit mit Quecksilber bei vorgegebenem Druck.

Es wurde im Rahmen der Erfindung festgestellt, daß bei bestimmten Regeneratcellulosen, wie der erfindungsgemäßen chemisch unmodifizierten Perlcellulose, in starkem Maße eine Bindung von LDL auftritt. Wesentliche Voraussetzung für diese Bindung der LDL ist, daß die in der Cellulosematrix befindlichen Makroporen auch zugänglich sind, d.h. einen bestimmten Mindesteingangsquerschnitt aufweisen. Überraschend hierbei ist jedoch, daß die wesentlich kleineren HDL und auch andere im Plasma vorhandene Proteine, die bei den erfindungsgemäßen Adsorbentien ebenfalls in stärkerem Maße in die Cellulosematrix eindringen können, nicht gebunden werden. Damit eine ausreichende Bindungskapazität für LDL erzielt wird, soll der Anteil an zugänglichen Makroporen einen Wert von 0,2 ml/g nicht unterschreiten. Höhere Werte begünstigen die Bindung und sollen erfindungsgemäß nach oben nicht begrenzt werden. Allerdings ergeben sich sowohl für das Gesamtporenvolumen in der Größenordnung von 2-5 ml/g als auch den Anteil an zugänglichen Makroporen in Höhe von 02-5 ml/g gewisse Grenzwerte, die durch das Herstellungsverfahren und die Neigung der Cellulose zur Ausbildung kompakter übermolekularer Strukturen bedingt sind. Außerdem wird bei einem Gesamtporenvolumen > 5 ml/g die Perlcellulose zunehmend mechanisch instabiler, so daß der Einsatz solcher Produkte in Suspension zwar noch geeignet, in Säulen aber nur bedingt möglich ist. Die Teilchengröße der erfindungsgemäßen Perlcelluloseprodukte kann im Bereich von 0,1 bis 3 mm liegen, wobei jedoch die Teilchengrößenfraktion von 0,1 bis 0,3 mm bevorzugt ist.

Zur Entfernung der LDL aus dem Blutplasma können die genannten Adsorbentien direkt im Plasma suspendiert oder als Packung in einer Säule angewendet werden, durch die das Plasma hindurchgepumpt wird. In beiden Fällen wird die wasserfeuchte Ausgangsperlcellulose mit den geforderten Porositätswerten zunächst mit 0,15 M NaCl-Lösung oder mit phosphatgepufferter NaCl-Lösung (PBS) bei einem pH-Wert von 7,4 gewaschen und überschüssige Flüssigkeit, z.B. durch Zentrifugieren, wieder weitgehend entfernt. Dann bringt man die feuchte Perlcellulose mit dem lipoproteinhaltigen Plasma in Kontakt. Das kann in einfacher Weise so erfolgen, daß man die Perlcellulose im Plasma suspendiert und unter leichtem Schütteln 10 bis 120 min beispielsweise bei Raumtemperatur oder 37 °C inkubiert und anschließend durch zentrifügieren wieder abtrennt. Dieses Verfahren findet vor allem bei analytischen und präparativen Arbeiten im klinischen Labor Anwendung, da auf diese Weise z.B. durch hohe Lipoproteingehalte getrübte Plasmen für analytische Untersuchungen geklärt oder LDL auch präparativ gewonnen werden können.

Für die Behandlung von Patienten mit familiärer Hypercholesterinämie wird die mit PBS vorbehandelte Perlcellulose in eine Säule oder auch andersartig gestaltete Vorrichtung gefüllt, die über einen Zu- und einen Ablauf verfügt, die mit plasmadurchlässigen Membranen verschlossen sind. Das Plasma wird dann durch die Perlcellulosepackung gepumpt, wobei der Durchsatz vorzugsweise 5 bis 50 ml/min beträgt. Die für eine ausreichende Bindung von LDL benötigte Menge Adsorbens beträgt für 1 l Plasma etwa 200 bis 500 g feuchte Perlcellulose. Ein großer Vorteil des erfindungsgemäßen Adsorbens besteht darin, daß es regenerierbar und damit mehrfach verwendungsfähig ist. Die Regenerierung kann in einfacher Weise durch aufeinanderfolgende Behandlung der mit LDL und Plasmaresten beladenen Perlcellulose mit 0,1 M Glycin-HCl-Puffer (pH 2,8), 5 bis 6 M Harnstofflösung und PBS erfolgen. Die regenerierten Produkte haben praktisch die gleiche Adsorptionskapazität wie beim erstmaligen Einsatz.

In den folgenden Beispielen werden erfindungsgemäß Adsorbentien und ihre Anwendung näher beschrieben.

## Beispiel 1

Wasserfeuchte Perlcellulose mit einem nach Verdrängung des Wassers durch Ethanol und Cyclohexan und Abschleudern auf einer Fritte bestimmten Cyclohexanrückhaltevermögen von 2,8 g/g Cellulose, entsprechend einem Gesamtporenvolumen von 3,59 ml/g, und einen quecksilberporosimetrisch bestimmten Porenvolumen von 0,38 ml/g für Poren mit einem Eintrittsquerschnitt von 20 bis 2000 nm wurde zunächst fünfmal mit der fünffachen Menge phosphatgepufferter 0,15 M NaCl-Lösung (pH 7,4) gewaschen und anschließend durch Zentrifugieren (5 min, 10000 m/s$^2$) sedimentiert. Nach Abdekantieren des Überstands wurde 1 g des Perlcellulosesediments, dessen Teilchengröße 0,15 bis 0,25 mm betrug, in ein Röhrchen eingewogen und mit 2 ml eines lipoproteinhaltigen Mischplasmas versetzt. Es folgten Inkubation bei 37 °C während 30 min im Schüttelwasserbad, Zentrifugation der Perlcellulose und Analyse des überstehenden Plasmas.

Die Proteinbestimmungen erfolgten nach der üblichen Methode der einfachen radialen Immundiffusion nach Mancini et al. Da Mischseren vieler Einzelspender verwendet wurden, d.h. annähernd Werte der Normalbereiche angenommen werden konnten, erschien es ausreichend, die Werte im Serumpool vor und nach Inkubation zu vergleichen und die letztgenannten als Relativwerte der erstgenannten in % anzugeben. In dieser Weise wurden auch die Apoproteine der LDL ($\beta$-Lipoprotein) und HDL ($\alpha_1$-Lipoprotein) bestimmt. Die klinisch-chemischen Werte der Lipoproteine wurden nach dem Arzneibuch der DDR, D.L., 1985, bestimmt. LDL-Cholesterin wurde unter der Voraussetzung, daß die Triglyceridwerte im Normbereich liegen, nach Friedewald, W.T. Levy, R.I. und Fredrickson, D.S., Clin. Chem. 18 (1972) 409-503, berechnet. Zur Ermittlung eines Kontrollbereiches wurden jeweils Albumin, Transferrin, $\overline{\alpha_2}$-Makroglobulin und die Immunglobuline A, G und M mitbestimmt, von denen bekannt ist, daß sie nicht selektiv gebunden werden. Eine selektive Bindung wird nur dann angenommen, wenn die Werte einer Substanz des Serums unterhalb des Kontrollbereiches liegen. Die erhaltenen Ergebnisse sind in Tabelle 1 zusammenfassend dargestellt (Probe 1/1).

Zum Vergleich wurde eine Perlcellulose mit gleicher Teilchengröße, aber einem Gesamtporenvolumen von 1 ,8 ml/g und einem Porenvolumen von 0,15 ml/g für Poren mit einem Eintrittsquerschnitt von 20 bis 2000 nm in der vorstehend beschriebenen Weise mit PBS äquilibriert und mit Serum inkubiert, worauf das Serum analysiert wurde. Die hierbei erhaltenen Ergebnisse sind ebenfalls in Tabelle 1 dargestellt (Probe 0).

Während bei der Probe 0 (Vergleichsprobe) kaum eine durch den Perlcellulosezusatz bedingte Herabsetzung der Werte für $\beta$-Lipoprotein, LDL- und Gesamt-Cholesterin zu verzeichnen ist, tritt bei der Probe 1/1 im Vergleich zur Probe 0 eine deutliche Erniedrigung dieser Werte auf, die auf eine selektive Adsorption der LDL zurückzuführen ist.

Bestimmungen der Komplementaktivitäten, des Fibrinogens und des Plasminogens zeigen keine bemerkenswerten Veränderungen nach Inkubationen von Serum oder Plasma mit Perlcellulose.

## Beispiel 2

Analog Beispiel 1 wurde eine Perlcellulose gleicher Teilchengröße, jedoch mit einem Gesamtporenvolumen von 2,5 ml/g und einem Porenvolumen von 0,27 ml/g für Makroporen mit einem Eintrittsquerschnitt von 20 bis 2000 nm (Probe 2) mit PBS vorbehandelt und mit Plasma in Kontakt gebracht. Die erhaltenen Ergebnisse sind ebenfalls in Tabelle 1 dargestellt.

Es wurde eine signifikante und selektive Adsorption der LDL erzielt.

## Beispiel 3

Die in Beispiel 1 zur Bindung von LDL bereits verwendete Perlcellulose (Probe 1/1) wurde zwecks Regenerierung zunächst einmal mit PBS gewaschen, danach dreimal mit jeweils der fünffachen Menge an 0,1 M Glycin-HCl-Puffer (pH 2,8) 15 min bei 37 °C im Schüttelwasserbad behandelt, dann dreimal in gleicher Weise mit 6 M Harnstofflösung inkubiert und schließlich dreimal mit jeweils der zehnfachen PBS-Menge gewaschen.

Die derart regenerierte Perlcellulose (Probe 1/2) wurde analog Beispiel 1 auf ihre LDL-Bindungsfähigkeit getestet. Die Ergebnisse sind in Tabelle 1 aufgeführt.

Nach der Inkubation wurde diese Probe ein weiteres Mal in der beschriebenen Weise regeneriert und mit Plasma in Kontakt gebracht. Die Ergebnisse sind ebenfalls in Tabelle 1 Probe 1/3) dargestellt. Sowohl die einmal als auch die zweimal regenerierte Perlcellulose zeigt praktisch die gleiche Lipoproteinadsorptionskapazität wie beim Ersteinsatz.

## Beispiel 4

Analog Beispiel 1 wurde Perlcellulose einer Teilchengröße von 0,2 bis 0,3 mm mit einem Gesamtporenvolumen von 4,2 ml/g und einem Porenvolumen von 0,45 ml/g für Poren mit einem Eintrittsquerschnitt von 20 bis 2000 nm (Probe 4/1) mit PBS vorbehandelt. 1 g dieses Adsorbens wurde in eine Säule gefüllt, deren Boden mit einer plasmadurchlässigen Membran verschlossen war. Anschließend wurden bei Raumtemperatur (22 °C) für die Dauer von 15 min 2, 4 bzw. 6 ml Plasma durch die Säule gepumpt. Nach Beendigung des Umpumpens erfolgte die Analyse des Blutplasmas wie in Beispiel 1 beschrieben. Die hierbei erhaltenen Ergebnisse sind in Tabelle 2, Spalte 4/1 dargestellt.

Spalte 4/2 enthält die jeweiligen Werte für dasselbe Produkt nach Regenerieren mit 25 ml Glycin-HCl-Puffer, 25 ml 5 M Harnstofflösung und 80 ml PBS.

Während bei beiden Proben die $\beta$-Lipoprotein-, GesamtCholesterin und LDL-Cholesterinwerte wiederum deutlich herabgesetzt sind, findet weder für $\alpha_1$-Lipoprotein und HDL-Cholesterin noch für die zweiwertigen Metallionen $Ca^{2+}$ und $Mg^{2+}$ eine merkliche Adsorption statt.

## Tabelle 1

Inkubation von Perlcelluloseproben unterschiedlicher
Porosität mit Blutplasma (30 min, 37 °C)

A d s o r p t i o n

in % der Plasmawerte vor Behandlung mit Perlcellulose

| Parameter | Probe 0 (Ver- gleichs- probe) | Probe 1/1 | Probe 1/2 | Probe 1/3 | Probe 2 |
|---|---|---|---|---|---|
| Albumin | $73\pm2$ | $78\pm4$ | $84\pm6$ | $74\pm3$ | $68\pm5$ |
| Transferrin | $78\pm10$ | $78\pm4$ | $88\pm6$ | $78\pm8$ | $72\pm4$ |
| $\alpha_2$-Makro- globulin | $76\pm5$ | $79\pm4$ | $90\pm2$ | $80\pm2$ | $70\pm4$ |
| $\beta$-Lipoprotein | $71\pm4$ | $24\pm5$ | $32\pm5$ | $23\pm5$ | $34\pm6$ |
| $\alpha_1$-Lipo- protein | $86\pm8$ | $66\pm2$ | $76\pm6$ | $66\pm4$ | $64\pm3$ |
| Gesamt-Chole- sterin | 68 | 21. | 34 | 23 | 32 |
| LDL-Cholesterin | 72 | 0 | 13 | 5 | 4 |
| Triglyceride | 76 | 55 | 43 | 44 | 68 |
| HDL-Cholesterin | 60 | 56 | 79 | 66 | 67 |
| Kontrollbe- reiche | 71-77-83 | | 71-81-91 | | 64-78-92 |

## Tabelle 2

Perfusion von 2, 4 bzw. 6 ml Blutplasma durch eine mit
1 g feuchter Perlcellulose gefüllte Säule (15 min, 22 °C)

| Parameter | Plasmamenge (ml) | Adsorption in % der Ausgangswerte Probe 4/1 | Probe 4/2 |
|---|---|---|---|
| β-Lipoprotein | 2 | $24 \pm 7$ | $24 \pm 7$ |
| | 4 | $41 \pm 3$ | $35 \pm 2$ |
| | 6 | $58 \pm 3$ | $53 \pm 1$ |
| $\alpha_1$-Lipoprotein | 2 | $73 \pm 2$ | $86 \pm 1$ |
| | 4 | $78 \pm 6$ | $95 \pm 1$ |
| | 6 | $88 \pm 3$ | $97 \pm 1$ |
| Gesamt-Cholesterin | 2 | 25 | 22 |
| | 4 | 42 | 37 |
| | 6 | 54 | 49 |
| LDL-Cholesterin | 2 | 1 | 0 |
| | 4 | 21 | 18 |
| | 6 | 32 | 30 |
| Triglyceride | 2 | 38 | 39 |
| | 4 | 88 | 57 |
| | 6 | 99 | 64 |
| HDL-Cholesterin | 2 | 70 | 90 |
| | 4 | 90 | 84 |
| | 6 | 90 | 95 |
| $Ca^{2+}$ | 2 | 100 | 83 |
| | 4 | 100 | 85 |
| | 6 | 135 | 85 |
| $Mg^{2+}$ | 2 | 74 | – |
| | 4 | 89 | – |
| | 6 | 93 | 85 |

## Patentansprüche

1.  Selektives Adsorbens zur Bindung von Lipoproteinen niedriger Dichte (LDL) auf der Basis von Perlcellulose,

**dadurch gekennzeichnet,** daß

das Adsorbens chemisch unmodifizierte Perlcellulose ist, die ein Gesamtporenvolumen von 2 bis 5 ml/g Trockenmasse aufweist, wobei der Anteil an zugänglichen Makroporen mit einem Eingangsquerschnitt von 20 bis 2000 nm mindestens 10 % des Gesamtporenvolumens beträgt.

2. Adsorbens nach Anspruch 1, dadurch gekennzeichnet, daß der Anteil an zugänglichen Makroporen ≧ 0,2 ml/g beträgt.

3. Adsorbens nach Anspruch 1 oder 2, dadurch gekennzeichnet, daß der Anteil an zugänglichen Makroporen 0,2 bis 5 ml/g beträgt.

4. Adsorbens nach einem der Ansprüche 1 bis 3, gekennzeichnet durch einen Teilchendurchmesser von 0,1 bis 3,0 mm.

5. Adsorbens nach einem der Ansprüche 1 bis 4, gekennzeichnet durch einen Teilchendurchmesser von 0,1 bis 0,3 mm.

6. Adsorbens nach einem der Ansprüche 1 bis 5, dadurch gekennzeichnet, daß die Perlcelluloseteilchen kugelförmig sind.

7. Verfahren zur selektiven Abtrennung von Lipoproteinen niedriger Dichte (LDL) aus Blutplasma oder Blutserum durch Inkontaktbringen des Blutplasmas mit einem Adsorbens auf der Basis von Perlcellulose und Abtrennung des beladenen Adsorbens vom Blutplasma,
gekennzeichnet durch

(A) Waschen wasserfeuchter, chemisch unmodifizierter Perlcellulose mit einem Gesamtporenvolumen von 2 bis 5 ml/g Trockenmasse, wobei der Anteil an zugänglichen Makroporen mit einem Eingangsquerschnitt von 20 bis 2000 nm mindestens 10 % des Gesamtporenvolumens beträgt, mit NaCl-Lösung oder phosphatgepufferter NaCl-Lösung (PBS),
(B) Abtrennung der gewaschenen Perlcellulose von der Waschflüssigkeit,
(C) Inkontaktbringen der abgetrennten Perlcellulose mit dem lipoproteinhaltigen Blutplasma und
(D) Abtrennung der LDL-beladenen Perlcellulose vom Blutplasma.

8. Verfahren nach Anspruch 7, dadurch gekennzeichnet, daß in Schritt A eine Perlcellulose mit einem Anteil an zugänglichen Makroporen ≧ 0,2 ml/g eingesetzt wird.

9. Verfahren nach Anspruch 7 oder 8, dadurch gekennzeichnet, daß in Schritt A eine Perlcellulose mit einem Anteil an zugänglichen Makroporen von 0,2 bis 5 ml/g eingesetzt wird.

10. Verfahren nach einem der Ansprüche 7 bis 9, dadurch gekennzeichnet, daß in Schritt A eine Perlcellulose mit einem Teilchendurchmesser von 0,1 bis 3,0 und vorzugsweise 0,1 bis 0,3 mm eingesetzt wird.

11. Verfahren nach einem der Ansprüche 7 bis 10, dadurch gekennzeichnet, daß in Schritt A die Perlcellulose mit 0,15 M NaCl-Lösung oder phosphatgepufferter NaCl-Lösung (pH 7,4) gewaschen wird.

12. Verfahren nach einem der Ansprüche 7 bis 11, dadurch gekennzeichnet, daß die Abtrennung der Perlcellulose von der Waschflüssigkeit in Schritt B bzw. vom Blutplasma in Schritt D durch Zentrifugieren vorgenommen wird.

13. Verfahren nach einem der Ansprüche 7 bis 12, dadurch gekennzeichnet, daß die Perlcellulose in Schritt C im Blutplasma suspendiert und die erhaltene Suspension bei einer Temperatur im Bereich von Raumtemperatur bis 37 ° C inkubiert wird.

14. Verfahren nach einem der Ansprüche 7 bis 12, dadurch gekennzeichnet, daß die Perlcellulose zumindest in Schritt C als Packung in einer Säule vorgesehen wird.

15. Verfahren nach einem der Ansprüche 7 bis 14, dadurch gekennzeichnet, daß die LDL nach Schritt D von der LDL-beladenen Perlcellulose desorbiert und gewonnen werden.

16. Verfahren nach einem der Ansprüche 7 bis 15, dadurch gekennzeichnet, daß das Adsorbens nach Schritt D durch Behandlung mit Glycin-HCl-Puffer sowie mit Harnstofflösung zur Wiederverwendung regeneriert wird.

17. Verwendung des Adsorbens nach einem der Ansprüche 1 bis 6 zur selektiven Abtrennung von Lipoproteinen niederer Dichte (LDL) aus Blutplasma.

**Claims**

1. Selective adsorbent based on pearl cellulose for binding low-density lipoproteins (LDL),
   **characterised in that**
   the adsorbent is chemically unmodified pearl cellulose with a total pore volume of 2 to 5 ml/g dry mass, wherein the proportion of accessible macropores with an entry cross-section of 20 to 2000 nm amounts to at least 10 % of the total pore volume.

2. Adsorbent according to claim 1, characterised in that the proportion of accessible macropores amounts to ≥ 0.2 ml/g.

3. Adsorbent according to claim 1 or 2, characterised in that the proportion of accessible macropores amounts to 0.2 to 5 ml/g.

4. Adsorbent according to one of claims 1 to 3, characterised by a particle diameter of 0.1 to 3.0 mm.

5. Adsorbent according to one of claims 1 to 4, characterised by a particle diameter of 0.1 to 0.3 mm.

6. Adsorbent according to one of claims 1 to 5, characterised in that the pearl cellulose particles are spherical.

7. Process for the selective separation of low-density lipoproteins (LDL) from blood plasma or blood serum by causing the blood plasma to come into contact with an adsorbent based on pearl cellulose and separation of the charged adsorbent from the blood plasma,
   characterised by
   (A) washing water-moist, chemically unmodified pearl cellulose having a total pore volume of 2 to 6 ml/g dry mass, wherein the proportion of accessible macropores with an entry cross-section from 20 to 2000 nm amounts to at least 10 % of the total pore volume, with NaCl solution or phosphate-buffered NaCl solution (PBS),
   (B) separating the washed pearl cellulose from the washing liquid,
   (c) causing the separated pearl cellulose to come into contact with the blood plasma containing lipoproteins,
   and
   (D) separating the pearl cellulose charged with LDL from the blood plasma.

8. Process according to claim 7, characterised in that in step A a pearl cellulose with a proportion of accessible macropores ≥ 0.2 ml/g is used.

9. Process according to claim 7 or 8, characterised in that in step A a pearl cellulose with a proportion of accessible macropores of 0.2 to 5 ml/g is used.

10. Process according to one of claims 7 to 9, characterised in that in step A a pearl cellulose with a particle diameter of 0.1 to 3.0 and preferably 0.1 to 0.3 mm is used.

11. Process according to one of claims 7 to 10, characterised in that in step A the pearl cellulose is washed with 0.15 M NaCl solution or phosphate-buffered NaCl solution (pH 7.4).

12. Process according to one of claims 7 to 11, characterised in that the pearl cellulose is separated from the washing liquid in step B or from the blood plasma in step D, by centrifuging.

**13.** Process according to one of claims 7 to 12, characterised in that in step C the pearl cellulose is suspended in the blood plasma, and the suspension obtained is incubated at a temperature within the range from room temperature to 37 ° C.

**14.** Process according to one of claims 7 to 12, characterised in that the pearl cellulose is provided, at least in step C, as a packing in a column.

**15.** Process according to one of claims 7 to 14, characterised in that the LDLs are desorbed and recovered from the LDL-charged pearl cellulose after step D.

**16.** Process according to one of claims 7 to 15, characterised in that the adsorbent is regenerated after step D by treatment with glycin-HCl-buffer and with urea solution for re-use.

**17.** Use of the adsorbent according to one of claims 1 to 6 for the selective separation of low-density lipoproteins (LDL) from blood plasma.

**Revendications**

**1.** Adsorbant sélectif pour la liaison de lipoprotéines de faible densité (LDL), à base de cellulose en perles, caractérisé en ce que l'adsorbant est une cellulose en perles non modifiée chimiquement, présentant un volume total de pores de 2 à 5 ml/g de masse sèche, la part de macropores accessibles ayant une section d'entrée de 20 à 2000 nm représentant au moins 10% du volume total des pores.

**2.** Adsorbant selon la revendication 1, caractérisé en ce que la part de macropores accessibles est supérieure ou égale à 0,2 ml/g.

**3.** Adsorbant selon la revendication 1 ou 2, caractérisé en ce que la part de macropores accessibles est de 0,2 à 5 ml/g.

**4.** Adsorbant selon l'une des revendications 1 à 3, caractérisé en ce que le diamètre des particules est de 0,1 à 3,0 mm.

**5.** Adsorbant selon l'une des revendications 1 à 4, caractérisé en ce que le diamètre des particules est de 0,1 à 0,3 mm.

**6.** Adsorbant selon l'une des revendications 1 à 5, caractérisé en ce que les particules de cellulose en perles sont sphériques.

**7.** Procédé de séparation sélective de lipoprotéines de faible densité (LDL) du plasma sanguin ou du sérum sanguin par mise en contact du plasma sanguin avec un adsorbant à base de cellulose en perles et séparation de l'adsorbant chargé du plasma sanguin, caractérisé par
(A) lavage de cellulose en perles humidifiée d'eau, non modifiée chimiquement, ayant un volume total de pores de 2 à 5 ml/g de masse sèche, la part de macropores accessibles ayant une section d'entrée de 20 à 2000 nm représentant au moins 10% du volume total des pores, avec une solution de NaCl ou une solution de NaCl tamponnée par du phosphate (PBS),
(B) séparation de la cellulose en perles lavée du liquide de lavage,
(c) mise en contact do la cellulose en perles séparée avec le plasma sanguin contenant les lipoprotéines,
et
(D) séparation de la cellulose en perles chargée de LDL du plasma sanguin.

**8.** Procédé selon la revendication 7, caractérisé en ce que l'on emploie dans l'étape A une cellulose en perles présentant une part de macropores acessibles supérieure ou égale à 0,2 ml/g.

**9.** Procédé selon la revendication 7 ou 8, caractérisé en ce qu'on emploie dans l'étape A une cellulose en parles présentant une part de macropores accessibles de 0,2 à 5 ml/g.

**10.** Procédé selon l'une des revendications 7 à 9, caractérisé en ce que l'on emploie dans l'étape A une cellulose en perles avec un diamètre des particules de 0,1 à 3,0 et de préférence de 0,1 à 0,3 mm.

11. Procédé selon l'une des revendications 7 à 10, caractérisé en ce que, dans l'étape A, on lave la cellulose en perles avec une solution 0,15 M de NaCl ou de NaCl tamponnée par du phosphate (pH 7,4).

12. Procédé selon l'une des revendications 7 à 11, caractérisé on ce que la séparation de la cellulose en perles du liquide de lavage dans l'étape B ou la séparation de la cellulose en perles du plasma sanguin dans l'étape D est effectuée par centrifugation.

13. Procédé selon l'une des revendications 7 à 12, caractérisé en ce que, dans l'étape C, la cellulose en perles est mise en suspension dans le plasma sanguin et que la suspension obtenue est incubée à une température allant de température ambiante jusqu'à 37°C.

14. Procédé selon l'une des revendications 7 à 12, caractérisé en ce que la cellulose en perles est prévue, du moins dans l'étape C, comme remplissage d'une colonne.

15. Procédé selon l'une des revendications 7 à 14, caractérisé an ce que, après l'étape D, les LDL sont désorbées de la cellulose en perles et récupérées.

16. Procédé selon l'une des revendications 7 à 15, caractérisé en ce que, après l'étape D, l'adsorbant est régénéré par traitement avec un tampon glycine-HC1 ainsi qu'avec une solution d'urée en vue de la réutilisation.

17. Utilisation de l'adsorbant selon l'une des revendications 1 à 6 pour la séparation sélective de lipoprotéines de faible densité (LDL) du sérum sanguin.